# EUROPEAN PATENT APPLICATION

(11) **EP 2 082 695 A1**
(43) Date of publication of application: **29.07.2009**
(21) Application number: 09000868.1
(22) Date of filing: 22.01.2009
(51) Int. Cl.: A61B 17/34, A61B 17/00, A61M 25/00

(54) **Injection needle for endoscope, and inner tube having needle body fixed to its distal end**

(30) Priority: 22.01.2008 JP 2008011329
(71) Applicant: FUJIFILM Corporation, Minato-ku Tokyo (JP)
(72) Inventor: Ohashi, Katsuaki, Tochigi-shi Tochigi (JP); Izaki, Toshihiko, Saitama-shi Saitama (JP); Iyama, Shozo, Saitama-shi Saitama (JP)
(74) Representative: Klunker . Schmitt-Nilson . Hirsch

(57) **Abstract**

An injection needle for an endoscope includes an insertion portion including, an outer tube, and an inner tube movably arranged forward and backward within the outer tube, and which has a coil member and a needle body fixed to a distal portion of the inner tube, and an operating portion which is fixed to the outer tube and for operating to move the inner tube forward and backward, wherein the coil member is fitted on and is anchored to a portion opposed to a tip of the needle body, and the inner tube is fitted on and anchored to the coil member.

## Description

The present invention claims priority from Japanese Patent Application No. 2008-011329 filed on January 22, 2008, the entire content of which is incorporated herein by reference.

### BACKGROUND OF INVENTION

### Field of the Invention

The present invention relates to an injection needle which is used by being inserted into a forceps channel of an endoscope, and particularly, relates to an injection needle for an endoscope which is suitable to inject a high-viscosity medicinal solution, and an inner tube having a needle body fixed to its distal end.

### Description of the Related Art

As a treatment instrument aiming at being inserted into a forceps channel of an endoscope to inject a medicinal solution under a mucosa of a digestive organ, there is an injection needle for an endoscope as described in, for example, JP-A-2001-58006.

This injection needle for an endoscope includes an elongate flexible insertion portion, and an operating portion provided on the proximal side of the insertion portion, and the insertion portion is inserted into a forceps channel of an endoscope. The insertion portion is composed of an outer tube and an inner tube, and the inner tube is disposed so as to be movable forward and backward within the outer tube. The injection needle is configured such that a needle body pierced into a mucosa of an alimentary canal is fixed to a distal end of the inner tube, and a medicinal solution is injected under an alimentary canal mucosa through the inner tube from the needle body.

When a low-viscosity medicinal solution, such as a saline solution, is injected by the injection needle for an endoscope, even if a thin inner tube whose internal diameter is the same as the external diameter of the needle body is used as the inner tube, the medicinal solution may be delivered. However, since high-viscosity medicinal solutions, such as a hyaluronate sodium solution, may not be injected unless conduit resistance is not reduced, it is effective to increase the internal diameter (liquid delivery portion) of the inner tube.

However, a thinner needle body is desirable if piercing performance for a mucosa is taken into consideration. For this reason, a clearance may be created between the internal diameter of the inner tube and the external diameter of the needle body, and a member which fills up the clearance between both is required.

Since stainless steel specified by JIS is used for the needle body, it is also preferable to make the member, which fills up the clearance between the needle body and the inner tube, of stainless steel.

On the other hand, it is necessary to manufacture the inner tube from a material having good sliding performance in order to make the inner tuber movable forward and backward within the outer tube. Typically, a tube formed of fluororesin known as Teflon (registered trademark) is used.

In fastening the inner tube made of fluororesin and the needle body made of stainless steel, fastening by press fitting is mainly used, and fastening by adhesion is also used supplementarily. In order to increase the fastening force by this adhesion, it is effective to form a number of grooves that become adhesive agent pools in a stainless material. However, if the grooves are formed in the needle body, the grooves are caught when the tip of the needle is polished. As a result, the polishing may become difficult, and manufacturing yield may be lowered.

In recent years, a high-viscosity hyaluronate sodium solution is more often used as the medicinal solution, and consequently, it necessary to use an inner tube with a large internal diameter so that the high-viscosity solution may flow easily. That is, the above member which fills up the clearance is required, and even if this member enters a gap between the needle body and the inner tube, a device to obtain the fastening force of JIS is required. Moreover, since an inner tube integral with a needle body which is used after being replaced with an injection needle for an endoscope is disposable in principle, the reduction of manufacturing cost is also required.

### SUMMARY OF INVENTION

An object of the present invention is to provide an injection needle for an endoscope which may be manufactured at low cost and may also inject a high-viscosity medicinal solution easily.

According to a first aspect of the invention, an injection needle for an endoscope includes an insertion portion including, an outer tube, and an inner tube movably arranged forward and backward within the outer tube, and which has a coil member and a needle body fixed to a distal portion of the inner tube, and an operating portion which is fixed to the outer tube and for operating to move the inner tube forward and backward, wherein the coil member is fitted on and is anchored to a portion opposed to a tip of the needle body, and the inner tube is fitted on and anchored to the coil member.

According to a second aspect of the invention, the coil member with a smaller internal diameter than an external diameter of the needle body is fitted on the needle body.

According to a third aspect of the invention, the inner tube with a smaller internal diameter than an external diameter of the coil member in a state of being fitted on the needle body is fitted on the coil member.

According to a forth aspect of the invention, a length of a portion of the coil member fitted on the needle body is at least 1/4 or more the length of the needle body.

According to a fifth aspect of the invention, each of the needle body and the coil member is made of stainless steel, and the inner tube is made of fluororesin.

According to a sixth aspect of the invention, the coil member is anchored to the needle body by welding or with an adhesive agent.

According to a seventh aspect of the invention, the inner tube is fitted on and anchored to the coil member by applying an adhesive agent to the coil member.

According to an eighth aspect of the invention, an inner tube includes a needle body fixed to a distal portion of the inner tube, and a coil member is fitted and is anchored to a portion opposed to a tip of the needle body, wherein the inner tube is replaceable for injection needle.

According to the present invention, it is possible to manufacture at low cost, it is possible to integrate a thin needle body with an inner tube with a large internal diameter by a required fastening force, and it is also possible to efficiently inject a high-viscosity medicinal solution without liquid leakage.

Other aspects and advantages of the present invention will be apparent from the following description and the appended claims.

### BRIEF DESCRIPTION OF THE DRAWINGS

Fig. 1 is a view showing an entire configuration of an injection needle for an endoscope according to one exemplary embodiment of the present invention;
Fig. 2 is an enlarged sectional view of a tip portion II of an insertion portion shown in Fig. 1;
Fig. 3 is a view showing an appearance of a distal end of an inner tube to which a needle body shown in Fig. 2 is fixed;
Fig. 4 is a side sectional view of the distal end of the inner tube shown in Fig. 3; and
Fig. 5 is an enlarged sectional view of a connecting portion V of the coil spring and the needle body, which is shown in Fig. 4.

### DESCRIPTION OF EXEMPLARY EMBODIMENTS

Hereinafter, one exemplary embodiment of the present invention will be described with reference to the accompanying drawings.

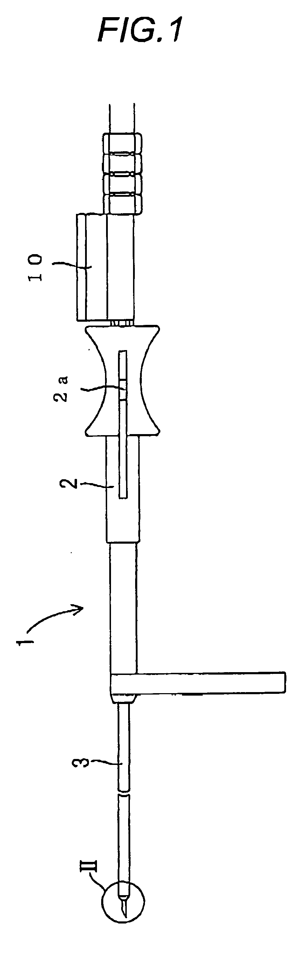

Fig. 1 is a view showing the entire configuration of an injection needle for an endoscope according to one exemplary embodiment of the present invention. The injection needle 1 for an endoscope according to this exemplary embodiment includes an operating portion 2, and an elongate flexible insertion portion 3 which projects from the operating portion 2.

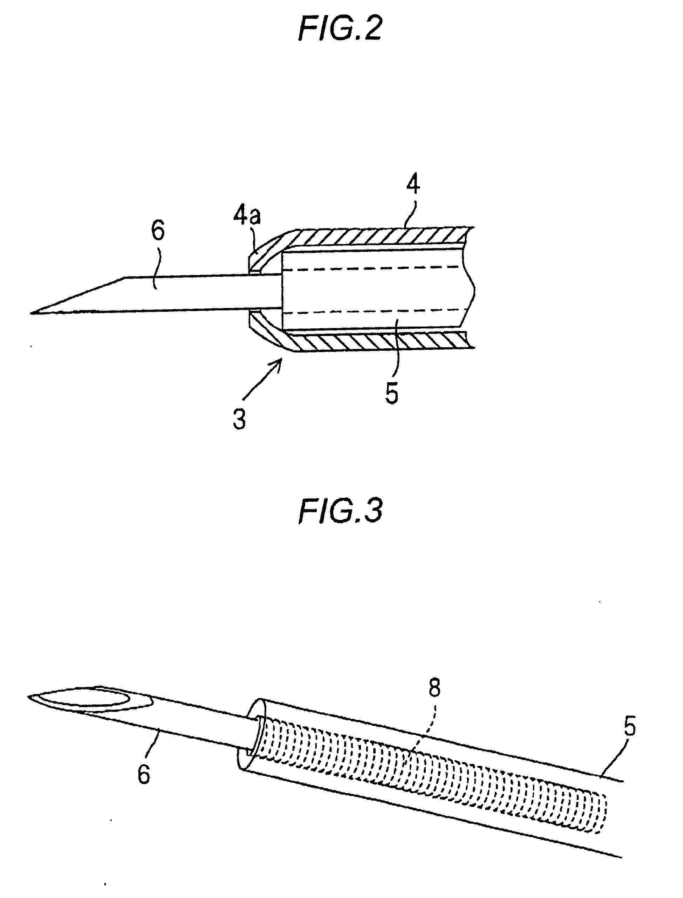

Fig. 2 is an enlarged sectional view of a tip portion II of the insertion portion 3 shown in Fig. 1. The insertion portion 3 includes an outer tube 4 having flexibility and fixedly connected to the operating portion 2, and an inner tube 5 disposed so as to be movable forward and backward within the outer tube 4, and integrally connected with a liquid feed pipe 2a of the operating portion 2 on the proximal side thereof.

By using a tube made of fluororesin as the inner tube 5, a needle body 6 is fixed to a distal end of the inner tube 5 as will be described in detail.

Although the diameter of a distal end 4a of the outer tube 4 is made smaller than the external diameter of the inner tube 5 and the needle body 6 protrudes to the outside, the inner tube 5 is throttled to such an internal diameter that the tube does not protrude to the outside. For this reason, when the inner tube 5 of the insertion portion 3 is operated to move forward and backward and is delivered toward the distal end 4a within the outer tube 4 in conjunction with sliding movement of the operating portion 2 shown in Fig. 1, only the needle body 6 protrudes to the outside and is pierced into an alimentary canal mucosa. Additionally, when the inner tube 5 is pulled back toward the operating portion 2, the needle body 6 is completely returned into the outer tube 4.

In addition, a member 10 shown adjacent to the operating portion 2 of Fig. 1 is a stopper during transportation, and is removed when the injection needle 1 for an endoscope is used.

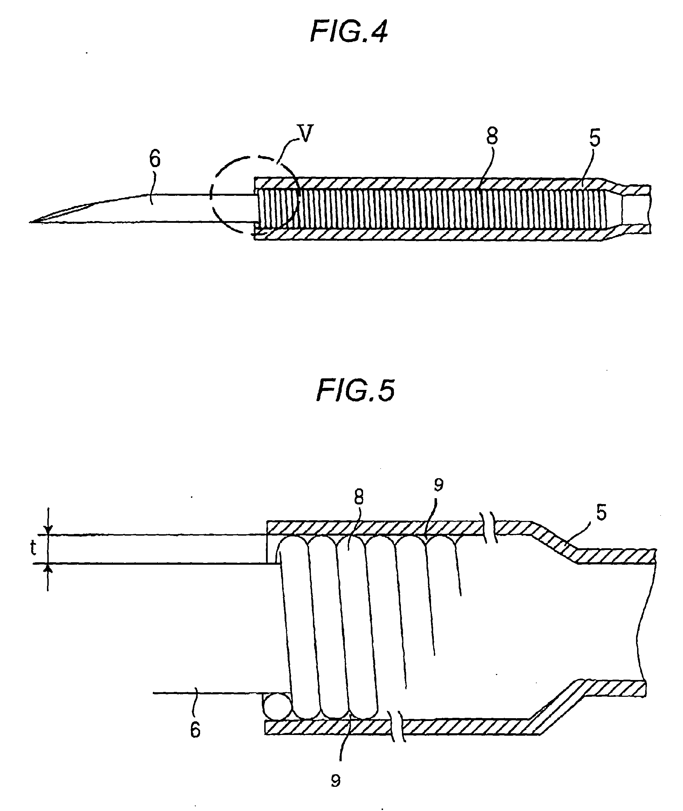

Fig. 3 is a perspective view of the appearance of the distal end of the inner tube 5 to which the needle body 6 is attached, Fig. 4 is a side view of the distal end of the inner tube, and Fig. 5 is an enlarged view of a portion V shown in Fig. 4.

A coil spring 8 made of stainless steel is fitted on an inner tube coupling portion of the needle body 6, and the inner tube 5 is fitted on an outer peripheral portion of the coil spring 8 so as to cover the total length of the coil spring 8.

As the coil spring 8, a coil spring whose internal diameter is slightly smaller than the external diameter of the needle body 6 is used. For this reason, when the coil spring 8 is fitted on the needle body 6, the coil spring 8 may fasten the outer periphery of the needle body 6 by its own resilience, and may improve the adhesion between the inner peripheral surface of the coil spring 8 and the outer peripheral surface of the needle body 6.

Additionally, as the coil spring 8 to be used in this exemplary embodiment, a coil spring having a length enough to cover at least 1/4 the total length (about 12 to 13 mm) of the needle body 6, preferably, a length enough to cover about half, and a length enough to cover about 60 percents in the illustrated example is used. Thereby, when the coil spring 8 is fitted on the needle body 6, the length that the outer peripheral surface of the needle body 6 and the inner peripheral surface of the coil spring 8 adhere to each other may be increased. As a result, the probability that a medicinal solution within the inner tube 5 may leak from a gap between both is eliminated.

In addition, since the exemplary embodiment shown is a preferred exemplary embodiment, the coil spring 8 is fitted on the needle body 6 such that the rear end surface position of the needle body 6 and the rear end surface position of the coil spring 8 may coincide with each other. However, there is no problem even if the rear end surface position of the needle body 6 projects slightly from the rear end surface position of the coil spring 8.

In a state where the coil spring 8 is fitted on the outer periphery of the needle body 6, both are strongly anchored to each other by laser welding, brazing, etc.

The inner tube 5 having a smaller diameter than the external diameter of the coil spring 8 in the state of being fitted on the needle body 6 is used as the inner tube 5, and the inner tube 5 is fitted on the coil spring 8. The coil spring 8 is fastened by the resilience of the inner tube 5, and the adhesion between the inner peripheral surface of the inner tube 5 and the outer peripheral surface of the coil spring 8 is increased. Since a long coil spring is used as the coil spring 8, the adhesion length becomes large, and the possibility that a medicinal solution in the inner tube 5 may leak from the gap between both the coil spring and the inner spring is eliminated.

When the inner tube 5 is fitted on the coil spring 8 fitted on the needle body 6, the inner tube is fitted on the coil spring by applying an adhesive agent to the coil spring 8. Since the coil spring 8 has several tens of spiral gaps at its outer peripheral surface, these gaps become adhesive agent pools 9. As a result, even if a tube made of fluororesin having a low adhesive property is used, it is possible to effectively stick the inner tube 5 and the coil spring 8 together.

According to the exemplary embodiment described above, it is possible to manufacture an injection needle for an endoscope capable of efficiently injecting a medicinal solution with no liquid leakage, using a coil spring that is an inexpensive ready-made article, or an inner tube made of fluororesin, without performing special machining on a stainless material.

Additionally, even if a coil spring manufactured by winding a thick stainless steel wire rod such that a clearance t (clearance between the outer periphery of the needle body 6 and the inner periphery of the inner tube 5) shown in Fig. 5 is increased is used, the fastening force between the inner tube 5 and the needle body 6 may be more than a required fastening force by attaching the needle body to the distal end of the inner tube by the above manufacturing method. Therefor, efficient injection of a high-viscosity medicinal solution, i,e., injection with little liquid leakage, is enabled using the thick inner tube 5, and a burden to a subject may be eased by using the thin needle body 6.

In addition, although the coil spring 8 is used in the above-described exemplary embodiment, the present invention is not limited thereto, and the coil spring may be substituted with other members, such as a spiral pipe formed by winding an elongate plate in the shape of a coil.

Additionally, it has been described that the needle body 6 and the coil spring 8 are welded together. However, by adopting the construction of the exemplary embodiment described above, it is possible to obtain a fastening force according to a JIS standard even if the needle body 6 and the coil spring 8 are fixedly stuck together not using welding but using a adhesive agent.

In this case, as a manufacturing procedure for the distal end of the injection needle, it is possible to adopt a procedure of fitting the coil spring into the distal end of the inner tube and integrating them, and then press-fitting the needle body into the coil spring, other than the procedure of fitting the coil spring on the needle body and fitting the inner tube on the coil spring. Since the welding becomes unnecessary, the manufacturing cost may be reduced much more.

Moreover, the coil spring 8 and the inner tube 5 are fastened together using an adhesive agent in the above-described exemplary embodiment. However, even if the adhesive agent is not used, it is possible to obtain a fastening force according to a JIS standard between both the coil spring and the inner tube only by press-fitting the coil spring onto the inner tube 5 because there are many grooves at the outer peripheral portion of the coil spring 8.

Since the injection needle according to the present invention may be easily manufactured, and may be realized at low cost, the injection needle is useful as an injection needle for an endoscope for injection of a high-viscosity medicinal solution.

## Claims

1. An injection needle for an endoscope comprising:
an insertion portion including:
an outer tube; and
an inner tube movably arranged forward and backward within the outer tube, and which has a coil member and a needle body fixed to a distal portion of the inner tube; and
an operating portion which is fixed to the outer tube and for operating to move the inner tube forward and backward,
wherein the coil member is fitted on and is anchored to a portion opposed to a tip of the needle body, and
the inner tube is fitted on and anchored to the coil member.

2. The injection needle for the endoscope according to Claim 1, wherein the coil member with a smaller internal diameter than an external diameter of the needle body is fitted on the needle body.

3. The injection needle for the endoscope according to Claim 1, wherein the inner tube with a smaller internal diameter than an external diameter of the coil member in a state of being fitted on the needle body is fitted on the coil member.

4. The injection needle for the endoscope according to Claim 1, wherein a length of a portion of the coil member fitted on the needle body is at least 1/4 or more the length of the needle body.

5. The injection needle for the endoscope according to Claim 1, wherein each of the needle body and the coil member is made of stainless steel, and the inner tube is made of fluororesin.

6. The injection needle for the endoscope according to Claim 1, wherein the coil member is anchored to the needle body by welding or with an adhesive agent.

7. The injection needle for the endoscope according to Claim 1, wherein the inner tube is fitted on and anchored to the coil member by applying an adhesive agent to the coil member.

8. An inner tube comprising:
a needle body fixed to a distal portion of the inner tube; and
a coil member is fitted and is anchored to a portion opposed to a tip of the needle body,
wherein the inner tube is replaceable for injection needle.
